# EUROPEAN PATENT SPECIFICATION

(11) **EP 1 546 152 B1**
(45) Date of publication and mention of the grant of the patent: **19.12.2007**
(21) Application number: 03747872.4
(22) Date of filing: 01.08.2003
(51) Int. Cl.: C07D 493/04, C07D 417/06, C07D 405/06, C07D 407/06, A61K 31/427, A61K 31/4427, A61P 35/00, A61P 17/06

(54) **EPOTHILONE DERIVATIVES**
EPOTHILONDERIVATE
DERIVES D'EPOTHILONE

(30) Priority: 02.08.2002 US 400535 P; 24.06.2003 US 480933 P
(43) Date of publication of application: 29.06.2005
(73) Proprietor: Novartis AG, 4056 Basel (CH); Novartis Pharma GmbH, 1230 Wien (AT); THE SCRIPPS RESEARCH INSTITUTE, La Jolla, CA 92037 (US)
(72) Inventor: NAMOTO, Kenji, 8006 Zürich (CH); NICOLAOU, Kyriacos, Costa, La Jolla, CA 92037 (US); RITZEN, Andreas, DK-2720 Vanlose (DK)
(74) Representative: Ott, Johann
(86) International application number: PCT/EP2003/008554
(87) International publication number: WO 2004/014919

(56) References cited:
- WO-A-98/25929
- WO-A-99/67252
- WO-A-03/018002
- NICOLAOU K.C. ET AL: "Chemical synthesis and biological evaluation of novel epothilone B and trans-12,13-cyclopropyl epothilone B analogues" TETRAHEDRON, vol. 58, no. 32, 2002, pages 6413-6432, XP002902983 ISSN: 0040-4020
- NICOLAOU K.C. ET AL: "Design Synthesis, and Biological Properties of Highly Potent Epothilone B Analogues" ANGEW. CHEM. INT. ED., vol. 42, no. 30, 4 August 2003 (2003-08-04), pages 3515-3520, XP002262410
- DATABASE STN INTERNATIONAL ALTMANN, KARL-HEINZ ET AL: "Synthetic and semisynthetic analogs of apothilones: chemistry and biological acitivity" Database accession no. 2002:132141 XP002262419 & ACS SYMPOSIUM SERIES (2001), 796 (ANTICANCER AGENTS), pages 112-130,
- NICOLAOU K C ET AL: "Chemical synthesis and biological properties of pyridine epothilones" CHEMISTRY & BIOLOGY, vol. 7, no. 8, 2000, pages 593-599, XP002262411 ISSN: 1074-5521
- NICOLAOU K C ET AL: "Chemical synthesis and biological evaluation of cis- and trans-12,13-cyclopropyl and 12,13-cyclobutyl epothilones and related pyridine side chain analogues" JOURNAL OF THE AMERICAN CHEMICAL SOCIETY, vol. 123, no. 38, 26 September 2001 (2001-09-26), pages 9313-9323, XP002262412 UNITED STATES ISSN: 0002-7863
- NICOLAOU K C ET AL: "Synthesis and biological evaluation of 12,13-cyclopropyl and 12,13-cyclobutyl epothilones" CHEMBIOCHEM: A EUROPEAN JOURNAL OF CHEMICAL BIOLOGY, vol. 2, no. 1, 8 January 2001 (2001-01-08), pages 69-75, XP002262413 GERMANY ISSN: 1439-4227
- NICOLAOU K.C. ET AL: "Chemical Biology of Epothilones" ANGEW. CHEM. INT. ED., vol. 37, no. 15, 1998, pages 2014-2045, XP002262414 ISSN: 1433-7851
- NICOLAOU K.C. ET AL: "Total synthesis of 16-desmethylepothilone B, epothilone B10, epothilone F, and related side chain modified epothilone B analogues" CHEMISTRY (WEINHEIM AN DER BERGSTRASSE, vol. 6, no. 15, 4 August 2000 (2000-08-04), pages 2783-2800, XP002262415 GERMANY ISSN: 0947-6539
- ALTMANN KARL-HEINZ ET AL: "Synthesis and biological evaluation of highly potent analogues of epothilones B and D" BIOORGANIC & MEDICINAL CHEMISTRY LETTERS, vol. 10, no. 24, 18 December 2000 (2000-12-18), pages 2765-2768, XP002262416 OXFORD ISSN: 0960-894X
- NICOLAOU KARL-HEINZ ET AL: "Synthesis and biological properties of C12,13-cyclopropylepothilone A and related epothilones" CHEMISTRY AND BIOLOGY, CURRENT BIOLOGY, vol. 5, no. 7, July 1998 (1998-07), pages 365-372, XP002262417 LONDON ISSN: 1074-5521
- JOHNSON J ET AL: "Synthesis, structure proof, and biological activity of epothilone cyclopropanes" ORGANIC LETTERS, vol. 2, no. 11, 1 June 2000 (2000-06-01), pages 1537-1540, XP002262418 UNITED STATES ISSN: 1523-1540

## Description

### Technical Field:

The invention relates to antitumor agents. More particularly, the invention related to analogs of epothilone B, trans-12,13-cyclopropyl epothilone B as antitumor agents.

### Summary:

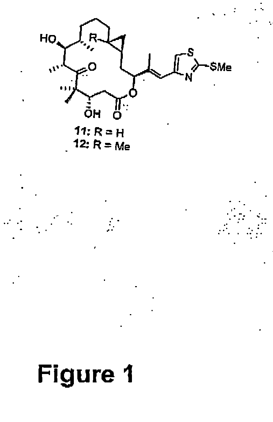

The invention is directed to analogs of epothilone B trans-12,13-cyclopropyl epothilone B having potent cytotoxic active against a variety of cell lines, including Taxol^{®}-resistant tumor cells. Exemplary embodiments of the invention include compound **12**, as illustrated in Figure 1, Another aspect of the invention is directed to the use of such compounds as cytotoxic agents.

One aspect of the invention is directed to a compound represented by formula I: 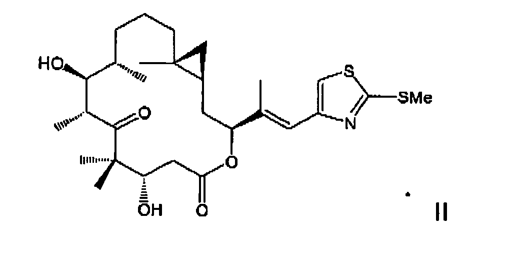 or a salt of a compound of formula I where a salt-forming group is present.

Where the plural form is used for compounds, salts, and the like, this is taken to mean also a single compound, salt, or the like ("a" as an indefinite article or as a numeral meaning "one").

Salts are primarily the pharmaceutically acceptable salts of compounds of formula I.

Such salts are formed, for example, as acid addition salts, preferably with organic or inorganic acids, from compounds of formula I with a basic nitrogen atom, especially the pharmaceutically acceptable salts. Suitable inorganic acids are, for example, hydrohalic acids, such as hydrochloric acid, sulphuric acid, or phosphoric acid. Suitable organic acids are, for example, carboxylic, phosphonic, sulphonic or sulphamic acids, for example acetic acid, propionic acid, octanoic acid, decanoic acid, dodecanoic acid, glycolic acid, lactic acid, 2-hydroxybutyric acid, gluconic acid, glucosemonocarboxylic acid, fumaric acid, succinic acid, adipic acid, pimelic acid, suberic acid, azelaic acid, malic acid, tartaric acid, citric acid, glucaric acid, galactaric acid, amino acids, such as glutamic acid, aspartic acid, N-methylglycine, acetylaminoacetic acid, N-acetylasparagine or N-acetylcysteine, pyruvic acid, acetoacetic acid, phosphoserine, 2- or 3-glycerophosphoric acid, maleic acid, hydroxymaleic acid, methylmaleic acid, cyclohexanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, glucuronic acid, galacturonic acid, methane- or ethanesulphonic acid, 2-hydroxyethanesulfonic acid, ethane-1,2-disulphonic acid, benzenesulphonic acid, 2-naphthalenesulphonic acid, 1,5-naphthalene-disulphonic acid, N-cyclohexylsulphamic acid, N-methyl-, N-ethyl- or N-propyl-sulphamic acid, or other organic protonic acids, such as ascorbic acid.

Salts of compounds of formula I with a salt-forming group may be prepared in a manner known per se. Acid addition salts of compounds of formula I may thus be obtained e.g. by treatment with an acid or with a suitable anion exchange reagent.

Salts can usually be converted to free compounds, e.g. by treating with suitable basic agents, for example with alkali metal carbonates, -hydrogencarbonates, or - hydroxides, typically potassium carbonate or sodium hydroxide.

For isolation or purification purposes it is also possible to use pharmaceutically unacceptable salts, for example picrates or perchlorates. Only the pharmaceutically acceptable salts or free compounds (if the occasion arises, in the form of pharmaceutical preparations) attain therapeutic use, and these are therefore preferred.

In view of the close relationship between the novel compounds in free form and in the form of their salts, including those salts that can be used as intermediates, for example in the purification or identification of the novel compounds, hereinbefore and hereinafter any reference to the free compounds is to be understood as referring also to the corresponding salts, as appropriate and expedient.

Another aspect of the invention is a process for synthesizing any of the compounds described above or intermediates thereof, as described in the specification, in particular a process for the preparation of a compound of formula I, II, III, IV or V wherein a compound of the formula VI 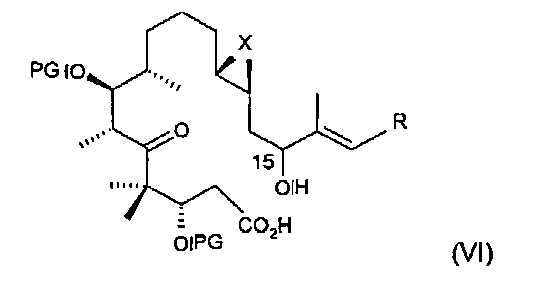 wherein X is CH₂ and R is 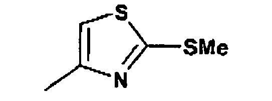 and PG is a protecting group for a hydroxy function,
in a first step is condensed by a esterification reaction, optionally in the presence of a catalyst,
and in a second step the protecting group is detached thus furnishing a lacton of formula I.
The term "protecting groups for a hydroxy group" as used herein refers to acid labile protecting groups for a hydroxy group, which groups are known as such. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products.The specialist knows, or can easily establish, which protecting groups are suitable with the reactions mentioned hereinabove and hereinafter.

The protection of hydroxy groups by protecting groups, the protecting groups themselves, and their cleavage reactions are described for example in standard reference works, such as J. F. W. McOmie, "Protective Groups in Organic Chemistry", Plenum Press, London and New York 1973, in T. W. Greene, "Protective Groups in Organic Synthesis", Wiley, New York 1981, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/l, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

Preferred protecting groups are silyl ethers which are acid labile like *tert*-butyldimethyl-silyl (TBS) ether, triethylsilyl (TES) ether, triisopropylsilyl (TIPS) ether, diethylisopropylsilyl (DEIPS) ether, isopropyldimethylsilyl (IPDMS) ether or thexyldimethylsilyl (TDS) ether.

### Brief Description of Drawings:

Figure 1 illustrates the structures of selected natural and designed epothilones. Grey boxes indicate compounds synthesized in this study.
Figure 4 illustrates a retrosynthetic analysis of *trans*-cyclopropyl epothilone B analogues (**12**
Figure 5 illustrates the construction of aldehyde **32.**
Figure 6 illustrates the construction of vinyl iodides **20e**.
Figure 7 illustrates the synthesis of epothilone analogues **12.**
Figure 8 illustrates a table disclosing the cytotoxicity of epothilones **1, 2** and **12** and paclitaxel against 1A9 human ovarian carcinoma cells and β-tubulin mutant cell lines selected with paclitaxel or epothilone A.
Figure 9 illustrates a table disclosing the tubulin polymerization potency and cytotoxicity of epothilone **1-2** and **12,** against human epidermoid cancer cell lines.
Figure 10 illustrates a table disclosing binding affinities of epothilone analogues to the taxoid binding site of microtubules.

Another aspect of the invention is directed to a pharmaceutical composition containing a therapeutic dose of a compound within either formula I, II, III, IV or formula V, represented above, for the treatment of a proliferative disease in a mammal. In a preferred mode, the mammal is a human.

### Detailed Description

The construction of a series of epoxide and cyclopropane epothilones with varying side chains by chemical synthesis and biologically evaluated is disclosed. The design of the present focused epothilone library was based on the current knowledge of structure activity relationships (SAR), specifically the facts that: (**1**) epothilone B **(2)** is considerably more potent than epothilone A (**1**); (2) a thiomethyl replacement for the methyl group on the thiazole moiety enhances the potency (Nicolaou, K. C.; et al. Angew. Chem. 1998, 110, 2120-2153; Angew. Chem. Int. Ed. 1998, 37, 2014-2045. Nicolaou, K. C.; et al. Tetrahedron 2002, 58, 6413-6432; Nicolaou, K. C.; et al. Angew. Chem. 1998, 110, 89-92; Angew. Chem. Int. Ed. 1998, 37, 84-87.); (3) a heterocycle such as pyridine (Nicolaou, K. C.; et al. Chem. Biol. 2000, 7, 593-599.) replacement for the thiazole ring needs to maintain the proper position for the nitrogen for biological activity; and (4) a cyclopropane ring can replace the epoxide moiety without loss of activity (Nicolaou, K. C.; et al. J. Am. Chem. Soc. 2001, 123, 9313-9323; Nicolaou, K. C.; et al. ChemBioChem. 2001, 2, 69-75; Johnson, J. A.; et al. Org. Lett. 2000, 2, 1537-1540.). From these considerations, epothilone **12** (figure 1) were considered as prime candidates for chemical synthesis and biological evaluation.The biological evaluation of these compounds led to the identification of the thiomethylthiazole side chain as a desirable pharmacophoric group improving the biological activity of the epothilones with regard to cytotoxicity and tubulin polymerizing properties. The enhanced activity was confirmed by three distinct biological assays where the effects of the compounds tested were determined both in cells and *in vitro.*

### Design and chemical synthesis of epothilone analogues:

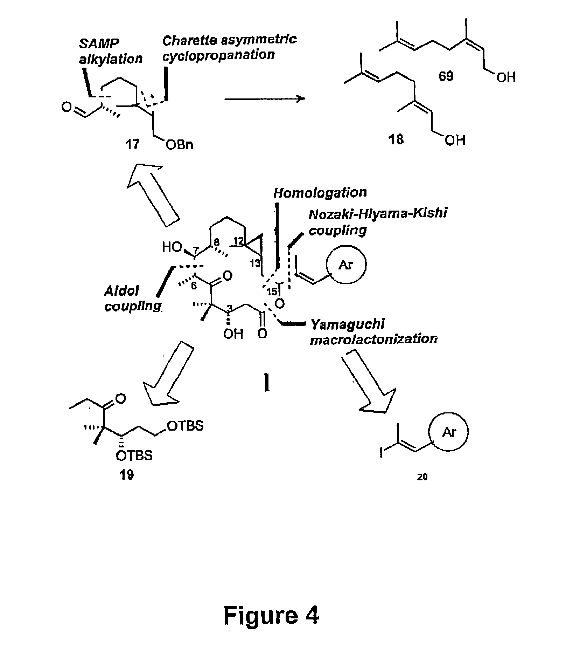

As an initial foray, we decided to confirm the potency enhancement bestowed on the epothilone scaffold by the methylthio group as compared to the methyl substituent in the epothilone **B** series. The methylthiothiazole epothilone **B (3)** was thus synthesized by Stille coupling of stannane **16** (Nicolaou, K. C.; et al. Bioorg. Med. Chem. 1999, 7, 665-697) with vinyl iodide **15** (Nicolaou, K. C.; et al. Chem. Eur. J. 2000, 6, 2783-2800) (80% yield) as shown in Figure 3. The observed high potency of analogue **3** against a series of tumor cell lines (see Table 1) encouraged us to proceed with the design and synthesis of an entire family of methylthio analogues as well as a number of new pyridine-containing epothilones.

Figure 4 outlines, in retrosynthetic format, the pathway that was followed for the construction of the cyclopropyl epothilone **B** analogues. Based on our previously reported strategy, the adopted sequence required a Charette cyclopropanation reaction (Nicolaou, K, C.; et al. J. Am. Chem. Soc. 2001, 123, 9313-9323; Charette, A. B.; et al. J. Am. Chem. Soc. 1998, 120, 11943-11952) to establish early on in the synthesis the 12,13-cyclopropyl site, an aldol reaction according to our optimized procedure (Nicolaou, K. C.; et al. Chem. Eur. J. 2000, 6, 2783-2800) to construct the C6-C7 bond with its two stereocenters, a Nozaki-Hiyama-Kishi coupling (Nicolaou, K. C.; et al. J. Am. Chem. Soc. 2001, 123, 9313-9323; Takai, K.; et al. Tetrahedron Lett. 1983, 24, 5281-5284; Jin, H.; et al. J. Am. Chem. Soc. 1986, 108, 5644-5646) to introduce the side chain, and a Yamaguchi macrolactonizaion (Inanaga, J.; et al. Bull. Chem. Soc. Jpn. 1979, 52, 1989-1993; Mulzer, J.; et al. Synthesis 1992, 215-228; Nicolaou, K. C.; et al. J. Am. Chem. Soc. 1997, 119, 7974-7991) to complete the macrocyclic structure. Key building blocks **18** or **69** and **19,** and **20** were thus defined as the starting points for these constructions. Construction of the corresponding epothilone A analogues was envisaged to be carried out in the same manner as previously reported by us (Nicolaou, K. C.; et al. J. Am. Chem. Soc. 2001, 123, 9313-9323).

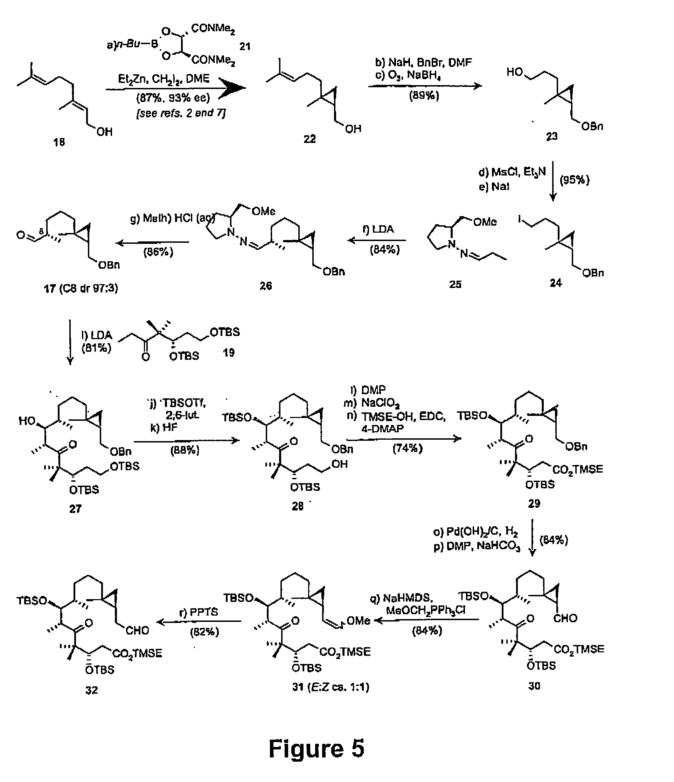

Figure 5 and figure 14 outline the synthesis of the required aldehyde **32** or **82** from the readily available geraniol **(18** or **69).** Thus, Charette cyclopropanation of **18** (Et₂Zn-CH₂l₂, in the presence of chiral ligand **21)** (Charette, A. B,; et al. J. Am. Chem. Soc. 1998, 120, 11943-11952) furnished cyclopropyl alcohol **22** in 87% yield and 93% ee or **71** in 80% yield, 95%ee. Protection of the hydroxy group in **22** or **71** (NaH-BnBr) (for abbreviations of reagents and protecting groups, see legends in schemes) followed by ozonolysis (O₃; NaBH₄) of the remaining double bond led to compound **23** or **72** in 89% or 83% overall yield respectively. Conversion of alcohol **23** or **72** to the corresponding iodide **(24,** 95% yield or **73,** 91 %) was accomplished upon mesylation and subsequent reaction with Nal. Alkylation of (-)-propionaldehyde SAMP hydrazone (**25**) (Nicolaou, K. C.; et al. J. Am. Chem. Soc. 1997, 119, 7974-7991; Enders, D. Aymmetric Synth. 1984, 3, 275-339; Enders, D.; Klatt, M. Synthesis 1996, 1403-1418) with iodide **24** or **73** under the influence of LDA gave compound **26** or **75** (84% yield, 87% yield respectively), whose cleavage (Mel; HCl_{aq}) led to aldehyde **17** in 86% yield or **76** in 91 % yield. The ratio of the resulting C-8 epimers was determined to be ca. 97;3 by ¹H NMR analysis of the MTPA esters derived from aldehyde **17** (Tsuda, M.; Endo, T.; Kobayashi, J. J. Org. Chem. 2000, 65, 1349-1352 and references cited therein). The aldol condensation between ketone **19** and aldehyde **17** or **76** under the previously defined conditions [LDA (2.4 equiv), ketone **19** (2.3 equiv), -78 to -40 °C, 30 min; then aldehyde **17** or **76,** -78 °C, 5 min] (Nicolaou, K, C.; et al. Chem. Eur, J. 2000, 6, 2783-2800) afforded aldol product **27** or **78** which was isolated in a diastereomerically pure form (81 % yield). Subsequent protection of the secondary alcohol in **27** or **78** as a TBS ether (TBSOTf, 2,6-lutidine) followed by selective cleavage of the primary TBS group (HF•py) afforded, in 88% overall yield, alcohol **28** or 86% overall yield, alcohol **79**. The compound was stepwise oxidized to the carboxylic acid (DMP; then NaClO₂) which was then protected as the TMSE ester **29** or **80**(TMSE-OH**,** EDC, 4-DMAP) in 75% or 73% overall yield. Hydrogenolysis of the benzyl ether in **29** or **80** followed by oxidation with DMP led to aldehyde **30** or **82** (84% yield, 87% yield) whose homologation (NaHMDS-MeOCH₂PPh₃Cl; then PPTS) to the coveted higher aldehyde **32** or **83** proceeded smoothly, and via vinyl ether **31** (ca. 1:1 E:Z ratio), with 82% overall yield.

The side chains (**20e,** Scheme 4) were synthesized from the corresponding aryl halides (**33** (Ellingboe, J. W.; et al. J. Med. Chem. 1994, 37, 542-550), **37**,) as shown in Scheme 4. Protection of 4-hydroxymethyl-2-pyridyl bromide 33 as a trityl ether (TrCl, 4-DMAP, 100%) followed by Sonogashira coupling (Arcadi, A.; et al. *Tetrahedron* **1994,** *50*, 437-452) of the resulting aryl bromide **34** with propyne [Pd(PPh₃)₂Cl₂-Cul, 96%] led to acetylenic compound **35** which served as a precursor to vinyl iodide **20c** (*n*-BuLi; then (*n*-Bu₃Sn)₂, CuCN, MeOH; then I₂, 80% yield). Exchange of the trityl for a MOM group within **35** [HCl(g), CHCl₃; then NaH, MOM-Cl, 34% overall yield] (Betzer, J.-F.; et al. Tetrahedron Lett. 1997, 38, 2279-2282**)** allowed access to vinyl iodide **20d** (67% yield) by exposure of the resulting intermediate **36** to the same conditions described above for the **35** to **20c** conversion. Similar chemistry was employed to construct vinyl iodide **20e** from **37,** respectively, as shown in Scheme 4.

Two crucial bond formations and two accompanying deprotections separated key building blocks **32** (prepared in this study for epothilone B analogues), **40** (prepared as previously described for epothilone A analogues) (Nicolaou, K. C.; et al. J. Am. Chem. Soc. 2001, 123, 9313-9323), and **20a-g** (for side chains) from the targeted epothilone analogues. The first operation was the Nozaki-Hiyama-Kishi coupling (Takai, K.; et al. Tetrahedron Lett. 1983, 24, 5281-5284; Jin, H.; et al. J. Am. Chem. Soc. 1986, 108, 5644-5646) of aldehydes **32** and **40** with vinyl iodides **20e.** This carbon-carbon bond forming reaction worked admirably in this instance (CrCl₂, NiCl₂, 4-*t*-BuPy, DMSO), furnishing, after TBAF-induced carboxylic acid generation, coupling products (**41e**,) in yields indicated in Schemes 5 (as ca. 1:1 mixtures of C-15 diastereomers). Each mixture of hydroxy acid diastereomers **(41e)** was then subjected to Yamaguchi macrocyclization (2,4,6-trichlorobenzoyl chloride, 4-DMAP) (Inanaga, J.; et al. Bull. Chem, Soc. Jpn. 1979, 52, 1989-1993; Mulzer, J.; et al. Synthesis 1992, 215-228) to afford the desired 15(S) lactone in the indicated (unoptimized) yields together with its 15(R) epimer. The separation of the two epimers at this juncture was facilitated by their rather drastically different R_{f} values on silica gel. Final deprotection of protected derivatives either with 20% TFA in CH₂Cl₂ **(43e),** led to epothilone 12 in the indicated (unoptimized) yields (Schemes 5). Chromatographically and spectroscopically pure compounds were subjected to biological evaluations as described below.

### Chemical biology:

The biological activities of the synthesized epothilones were evaluated through cytotoxicity, *in vitro* tubulin polymerization, and tubulin binding assays. Cytotoxicity was first evaluated in a set of ovarian carcinoma cell lines, including a parental cell line (IA9) and three drug-resistant cell lines, namely the paclitaxel-resistant strains (Giannakakou, P.; et al. J. Biol. Chem. 1997, 272, 17118-17125) IA9/PTX10 and IA9/PTX22 and the epothilone-resistant strain (Giannakakou, P.; et al. Proc. Natl. Acad. Sci. U.S.A. 2000, 97, 2904-2909) 1A9/A8. These resistant cell lines harbor distinct acquired β-tubulin mutations which affect drug-tubulin interaction and result in impaired taxane and epothilone-driven tubulin polymerization. The results of these biological investigations are summarized in Table 1 (Skehan, P.; et al. J. Natl. Cancer Inst. 1990, 82, 1107-1112). Further cytotoxicity and *in vitro* tubulin polymerization assays were carried out using a set of human epidermoid cancer cell lines, including a parent cell line (KB-31) and a paclitaxel-resistant (due to Pgp overexpression) cell line (KB-8511). The results of these studies are summarized in Table 2 (Nicolaou, K. C.; et al. Chem. Biol. 2000, 7, 593-599; Meyer, T.; et al. Int. J. Cancer 1989, 43, 851-856).

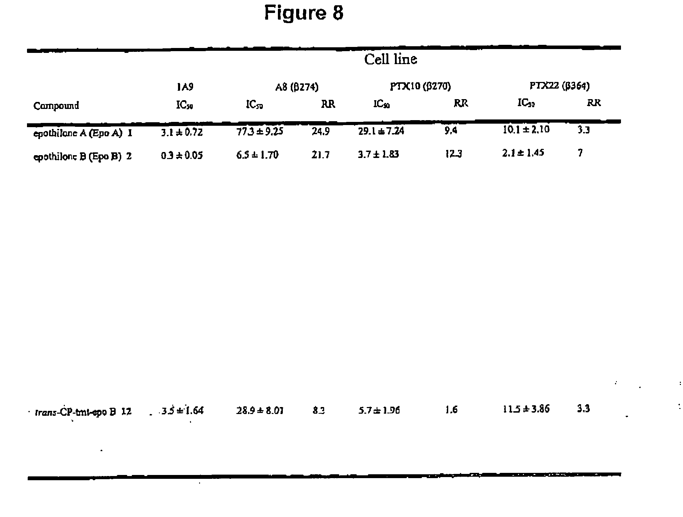

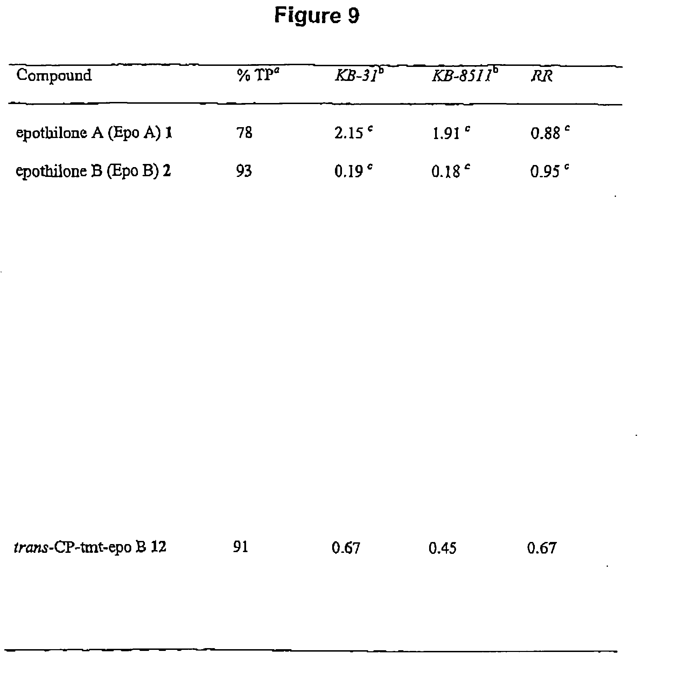

In general, there is good agreement between the *in vitro* tubulin polymerization potency and the cytotoxicity profile of the tested compounds against both the 1A9 human ovarian carcinoma cells and the KB-31 human epidermoid carcinoma cells. In agreement with original observations with the naturally occurring epothilones A and B, none of the epothilone A or B analogues tested herein appears to be a good substrate for the drug-efflux pump P-glycoprotein (Pgp). This is evident by the lack of cross-resistance of each of these analogues to the Pgp expressing cell line KB-8511, in contrast to paclitaxel-a known Pgp substrate- which is 214-fold less active against KB-8511 cells (see figure 9 and figure 17). It is noteworthy that all the epothilone analogues appear more active against the β-tubulin mutants compared to epothilone A (1) and epothilone B (2) (see figure 8 and figure 16, RR values). This is more pronounced with compound 12 for which the relative resistance values (RR) range from 1.6-9.3 against PTX10 (β270) and A8 (β274) cells compared with 9.4-24.9 RR values for Epo A (1) and Epo B (2) (figure 8). Furthermore, in the current study, and in agreement with previous reports (Nicolaou, K. C.; et al. ChemBioChem 2001, 2, 69-75; Giannakakou, P.; et al. J. Biol. Chem. 1997, 272, 17118-17125; Giannakakou, P.; et al. Proc. Natl. Acad. Sci. U.S.A. 2000, 97,2904-2909), we found that the paclitaxel-selected mutant PTX22 (β364) retains almost full sensitivity to the epothilones, and to all epothilone analogues tested in this report (RR values æ 3.3).

There is a general agreement in the relative potency of the substituted epothilone B analogs against the 1A9 human ovarian and the KB-31 human epidermoid cancer cells. Collectively, the results of these cytotoxicity assays revealed interesting information in terms of structure-activity relationships within the epothilone family. First, compounds 4 and 6 in which the C12-C13 epoxide moiety is replaced by a cyclopropane ring are the two most potent compounds among all the epothilone B analogs presented here. This result reaffirms that the C12-C13 epoxide moiety is not necessary for biological activity as previously noted (Nicolaou, K. C.; et al. J. Am. Chem. Soc. 2001, 123, 9313-9323; Nicolaou, K. C.; et al. ChemBioChem. 2001, 2, 69-75; Johnson, J. A.; et al. Org. Lett. 2000, 2, 1537-1540.). Compound 4 is 6-fold more active than the parent epothilone B (2) against the 1A9 human ovarian carcinoma cells (Figure 8) further confirming that the replacement of the methyl group on the thiazole side-chain with a thiomethyl group leads to increased activity. This result is in agreement with previous data on a similar substitution in epothilone B without replacement of the C12-C13 epoxide (i.e. compound 3) (Nicolaou, K. C.; et al. Tetrahedron 2002, 58, 6413-6432). The latter compound (3) was about 2-fold more active than the parent epothilone B, while compound 4 is 6-fold more potent than epothilone B. This result makes compound 4, the most active epothilone B analog against the 1A9 cell line synthesized to date and suggests that replacement of the epoxide by a cyclopropane moiety together with the replacement of the methyl substituent on the thiazole moiety with a thiomethyl group act synergistically, leading to the observed enhancement of biological activity. Interestingly, substitution of the methyl group of the thiazole ring with larger moieties (compound 12) led to diminished biological activity as compared to epothilone B (Figures 8 and 9).

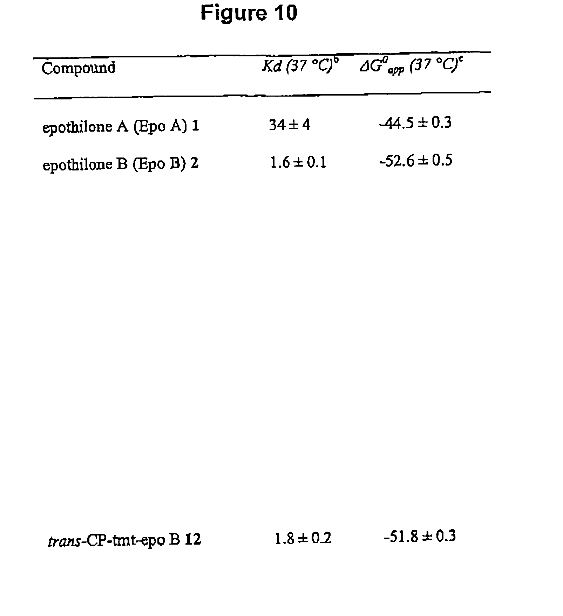

In addition to the above biological assays, the relative potency of each epothilone analogue was measured by the fluorescent taxoid displacement assay (Andreu, J. M.; Barasoain, I. Biochemistry 2001, 40, 11975-11984). The purpose of these experiments was to compare the equilibrium constants with which microtubules bind at their taxane site the epothilone analogues investigated. The inhibition of the binding of the well-characterized fluorescent taxoid Flutax-2 (Souto, A. A.; et al. Angew. Chem. Int. Ed, Engl. 1995, 34, 2710-2712; Diaz, J. F.; et al. J. Biol. Chem. 2000, 275, 26265-26276: Abal, M.; et al. Cell. Motil. Cytoskeleton 2001, 49, 1-15) to microtubules by each of the epothilone analogues was measured at 37 °C (Figure 10). The resulting equilibrium dissociation constants shown in Table indicate that epothilone A (1) has the lowest binding affinity among the epothilone analogues tested (Kd = 34Å4). The most powerful ligand among those measured in this assay is compound 3, with a Kd value of 0.64Å0.24 nM, followed by compound 12, with similar Kd value n 1.8 nM . the binding affinities of the analogues tested mirror their respective activities in both cell growth inhibition and *in vitro* tubulin polymerization assays.

Collectively from all three biological assays employed herein, a number of conclusions can be drawn in terms of structure-activity relationships within the epothilone family. First, the addition of the C12 methyl group does not enhance the activity in the trans-cyclopropyl series (compound 5 vs 6, 7 vs 8, 9 vs 10), contrary to the result in the cis epoxide series, where epothilone B (2) is at least 10-fold more active than epothilone A (1). This could be due to the different orientation of the C12 methyl group in the cis and trans compounds or to overall differences in conformation between the cis and trans compounds, although the details remain to be elucidated. Second, the introduction of the 2-thiomethylthiazole side chain enhances the activity compared with the natural 2-methylthiazole side chain (compounds 2 vs 3, 5 vs 11, and 6 vs 12). This effect was previously observed for epothilone C and D analogues (Nicolaou, K. C.; et al. Angew. Chem. 1997, 109, 2181-2187; Angew, Chem. Int. Ed. Engl. 1997, 36, 2097-2103; see also: Sinha, S. C.; et al. ChemBioChem 2001, 2, 656-665). Third, the replacement of a methyl group with a thiomethyl group in the pyridine side chain series (compounds 8 vs 13) reduces potency, contrary to the results obtained for the thiazole side chains above. This conclusion was based on the cell cytotoxicity and in vitro tubulin polymerization data, while in the fluorescent taxoid displacement assay the replacement of the methyl group with a thiomethyl moiety in the pyridine side chain is indifferent in terms of binding affinity. This discrepancy may simply reflect differences in cell uptake and permeability of the compounds tested or differences in the sensitivity of the two tubulin assays. Despite this discrepancy, it is clear from these data that the introduction of a thiomethyl group at the thiazole side chain is a more favorable modification than the introduction of a thiomethyl group at the pyridine side chain, which may be due to differing steric requirements by the two side chain scaffolds. In agreement with previous data obtained with cis pyridine epothilone analogues (Nicolaou, K. C.; et al. Chem. Biol. 2000, 7, 593-599), relocation of the thiomethyl group of the pyridine side chain from the position 5 (compound 13) to position 6 (compound 14) resulted in significant loss of activity. Fourth, mixed results are obtained with compounds 7 vs 9 and 8 vs 10 in which the 5-methylpyridine side chain (compounds 7 and 8) is substituted by the 5-hydroxymethylpyridine side chain (compounds 9 and 10). This substitution appears indifferent in cytotoxicity assays against the 1A9 human ovarian carcinoma cells (Table 1) where very similar IC50 values are obtained for each pair (e.g. 0.6 and 0.7 nM for compounds 7 and 9, respectively; 1.7 nM for compounds 8 and 10). On the other hand, in the human epidermoid carcinoma cells KB-31, compound 10 is 2-fold more active than its counterpart compound 8 with IC50s at 0.44 vs 0.9 nM, respectively. Given the small differences in the growth rate of the two human cancer cell lines that could account for the differential results, we could conclude that the introduction of the 5-hydroxymethylpyridine side chain is not likely to enhance activity in, at least, trans-12,13-cyclopropyl analogues of the epothilone family.

Owing to these properties, the compounds are suitable for the treatment of proliferative diseases, especially tumour diseases, including metastases; for example solid tumours such as lung tumours, breast tumours, colorectal tumours, prostate tumours, melanomas, brain tumours, pancreas tumours, neck tumours, bladder tumours, neuroblastomas, throat tumours, but also proliferative diseases of blood cells, such as leukaemia; also for the treatment of other diseases which respond to treatment with microtubule depolymerisation inhibitors, such as psoriasis.

A compound of formula I can be administered alone or in combination with one or more other therapeutic agents, possible combination therapy taking the form of fixed combinations or the administration of a compound of the invention and one or more other therapeutic agents being staggered or given independently of one another, or the combined administration of fixed combinations and one or more other therapeutic agents. A compound of formula I can besides or in addition be administered for tumour therapy in combination with chemotherapy, radiotherapy, immunotherapy, surgical intervention, or a combination of these. Long-term therapy is equally possible as is adjuvant therapy in the context of other treatment strategies, as described above. Other possible treatments are therapy to maintain the patient's status after tumour regression, or even chemopreventive therapy, for example in patients at risk.

Therapeutic agents for possible combination are especially one or more antiproliferative, cytostatic or cytotoxic compounds, for example one or more chemotherapeutic agent(s) selected from the group comprising the classical chemotherapeutic agents, an inhibitor of polyamine biosynthesis, an inhibitor of protein kinase, especially of serine/threonine protein kinase, such as protein kinase C, or of tyrosine protein kinase, such as epidermal growth factor receptor protein tyrosine kinase, a cytokine, a negative growth regulator, such as TGF-β or IFN-β, an aromatase inhibitor, and a classical cytostatic.

Compounds according to the invention are not only for the (prophylactic and preferably therapeutic) treatment of humans, but also for the treatment of other warm-blooded animals, for example of commercially useful animals, for example rodents, such as mice, rabbits or rats, or guinea-pigs. They may also be used as a reference standard in the test systems described above to permit a comparison with other compounds.

A compound of formula I may also be used for diagnostic purposes, for example with tumours that have been obtained from warm-blooded animal "hosts", especially humans, and implanted into mice to test them for decreases in growth after treatment with such a compound, in order to investigate their sensitivity to the said compound and thus to improve the detection and determination of possible therapeutic methods for neoplastic diseases in the original host.

Stereoisomeric mixtures, e.g. mixtures of diastereoisomers, can be separated into their corresponding isomers in a manner known per se by means of suitable separation methods. Diastereoisomeric mixtures may thus be separated into their individual diastereoisomers by means of fractionated crystallization, chromatography, solvent distribution, and similar procedures. This separation may take place either at the stage of one of the starting compounds or in a compound of formula I itself. Enantiomers may be separated through the formation of diastereoisomeric salts, for example by salt formation with an enantiomer-pure chiral acid, or by means of chromatography, for example by HPLC, using chromatographic substrates with chiral ligands. (Enantiomer separation is normally effected at the intermediate stage).

### Pharmaceutical preparations, methods, and uses

The present invention relates also to pharmaceutical preparations that contain a compound of formula I as active ingredient and that can be used especially in the treatment of the diseases mentioned above. Preparations for enteral administration, such as nasal, buccal, rectal or, especially, oral administration, and for parenteral administration, such as intravenous, intramuscular or subcutaneous administration, to warm-blooded animals, especially humans, are especially preferred. The preparations contain the active ingredient alone or, preferably, together with a pharmaceutically acceptable carrier. The dosage of the active ingredient depends upon the disease to be treated and upon the species, its age, weight, and individual condition, the individual pharmacokinetic data, and the mode of administration.

The precise dosage of the compounds of formula I to be employed for inhibiting proliferative disease, preferably tumors, depends upon several factors including the host, the nature and the severity of the condition being treated, the mode of administration and the particular compound employed. However, in general, satisfactory inhibition of tumors is achieved when a compound of formula I is administered parenterally, e.g., intraperitoneally, intravenously, intramuscularly, subcutaneously, intratumorally, or rectally, or enterally, e.g., orally, preferably intravenously or orally, more preferably intravenously at a single dosage of 1-300 mg/kg body weight per cycle (cycle = 3-6 weeks) or, for most larger primates, a single dosage of 50-5000 mg per treatment cycle. A preferred intravenous single dosage per 3-6 week treatment cycle is 1-75 mg/kg body weight or, for most larger primates, a daily dosage of 50-1500 mg. A typical intravenous dosage is 45 mg/kg, once every three weeks.

Usually, a small dose is administered initially and the dosage is gradually increased until the optimal dosage for the host under treatment is determined. The upper limit of dosage is that imposed by side effects and can be determined by trial for the host being treated.

The invention relates also to pharmaceutical preparations for use in a method for the prophylactic or especially therapeutic treatment of the human or animal body, to a process for the preparation thereof (especially in the form of compositions for the treatment of tumours) and to a method of treating the above-mentioned diseases, primarily neoplastic diseases, especially those mentioned above.

The invention relates also to processes and to the use of compounds of formula I for the preparation of pharmaceutical preparations which contain compounds of formula I as active component (active ingredient).

Preference is given to a pharmaceutical composition that is suitable for administration to a warm-blooded animal, especially a human or commercially useful mammal, suffering from a disease that is responsive to the inhibition of microtubule depolymerisation, for example psoriasis or especially a neoplastic disease, comprising a correspondingly effective amount of a compound of formula I, or a pharmaceutically acceptable salt thereof when salt-forming groups are present, together with at least one pharmaceutically acceptable carrier.

A pharmaceutical composition for the prophylactic or especially therapeutic treatment of neoplastic and other proliferative diseases of a warm-blooded animal, especially a human or a commercially useful mammal requiring such treatment, especially suffering from such a disease, comprising a new compound of formula I, or a pharmaceutically acceptable salt thereof, as active ingredient in a quantity that is prophylactically or especially therapeutically active against said diseases, is likewise preferred.

Pharmaceutical preparations contain from about 0.000001 % to 95 % of the active ingredient, whereby single-dose forms of administration preferably have from approximately 0.00001 % to 90 % and multiple-dose forms of administration preferably have from approximately 0.0001 to 0.5 % in the case of preparations for parenteral administration or 1 % to 20 % active ingredient in the case of preparations for enteral administration. Unit dose forms are, for example, coated and uncoated tablets, ampoules, vials, suppositories or capsules. Further dosage forms are, for example, ointments, creams, pastes, foams, tinctures, lipsticks, drops, sprays, dispersions, etc. Examples are capsules containing from about 0.0002 g to about 1.0 g active ingredient.

The pharmaceutical preparations of the present invention are prepared in a manner known per se, for example by means of conventional mixing, granulating, coating, dissolving or lyophilising processes.

The formulations suitable for parenteral administration are primarily aqueous solutions ([or example in physiological saline, obtainable by diluting solutions in polyethylene glycol, such as polyethylene glycol (PEG) 300 or PEG 400] of an active ingredient in water-soluble form, e.g. a water-soluble salt, or aqueous injectable suspensions containing viscosity-increasing agents, e.g. sodium carboxymethyl cellulose, sorbitol and/or dextran, and where appropriate stabilisers. The active ingredient, if need be together with excipients, can also be in the form of a lyophilisate and can be made into a solution before parenteral administration by the addition of suitable solvents.

Solutions such as those used, for example, for parenteral administration can also be employed as infusion solutions.

The invention similarly relates to a process or a method for the treatment of one of the above-mentioned pathological conditions, especially a disease which responds to an inhibition of microtubule depolymerisation, especially a corresponding neoplastic disease. A compound of formula I can be administered as such or in the form of pharmaceutical compositions, prophylactically or therapeutically, preferably in an amount effective against the said diseases, to a warm-blooded animal, for example a human, requiring such treatment, the compounds especially being used in the form of pharmaceutical compositions. In the case of an individual having a bodyweight of about 70 kg the dose administered is from approximately 0.1 mg to approximately 1 g, preferably from approximately 0.5 mg to approximately 200 mg, of a compound of the present invention. Administration is preferably effected e.g. every 1 to 4 weeks, for example weekly, every two weeks, every three weeks or every 4 weeks.

The present invention also relates in particular to the use of a compound of formula I, or a pharmaceutically acceptable salt thereof, especially a compound of formula I named as a preferred compound, or a pharmaceutically acceptable salt thereof, as such or in the form of a pharmaceutical formulation containing at least one pharmaceutically employable carrier, for the therapeutical and also prophylactic treatment of one or more of the above diseases.

The present invention also relates in particular to the use of a compound of formula I, or a pharmaceutically acceptable salt thereof, especially a compound of formula I named as a preferred compound, or a pharmaceutically acceptable salt thereof, for the preparation of a pharmaceutical formulation for the therapeutical and also prophylactic treatment of one or more of the above diseases.

### Experimental

### General

All reactions were carried out under an argon atmosphere with dry solvents under anhydrous conditions, unless otherwise noted. Anhydrous solvents were obtained by passing them through commercially available activated alumina columns. All reagents were purchased at highest commercial quality and used without further purification. Reactions were generally monitored by thin-layer chromatography carried out on 0.25 mm E. Merck silica gel plates (60F-254). E. Merck silica gel (60, particle size 0.040-0.063 mm) was used for flash column chromatography. Preparative thin-layer chromatography (PTLC) separations were carried out on 0.25, 0.50 or 1 mm E. Merck silica gel plates (60F-254). Melting points (mp) are uncorrected and were recorded on a Thomas-Hoover Unimelt capillary melting point apparatus. Optical rotations were recorded on a Perkin-Elmer 241 polarimeter. NMR spectra were recorded on Bruker DRX-600, DRX-500, AMX-400 or AC-250 instruments and calibrated using residual undeuterated solvents as an internal reference. All labeling of carbon atoms, e.g. C15, refers to epothilone A (1) numbering (see Figure 1). IR spectra were recorded on a Perkin-Elmer 1600 series FT-IR spectrometer. High resolution mass spectra were recorded on a PerSeptive Biosystems Voyager™ IonSpec mass spectrometer (MALDI-FTMS) or on an API 100 Perkin-Elmer mass spectrometer (ESI).

### Synthesis of epothilone 3

### Stille coupling of vinyl iodide 15 with stannane 16.

A solution of Pd₂(dba)₃(CHCl₃ (3.9 mg, 3.8 µmol), AsPh₃ (4.6 mg, 15 µmol), and Cul (7.2 mg, 38 µmol) in DMF (degassed, 0.5 mL) was added at 25 °C to a solution of iodide **15** (10 mg, 19 µmol) (Nicolaou, K. C., et al., Chem. Eur. J. 2000, 6, 2783-2800) and stannane **16** (11 mg, 38 µmol) (Nicolaou, K. C., et al., Bioorg. Med. Chem. 1999, 7, 665-697) in DMF (degassed, 0.5 mL), and the resulting solution was stirred for 2 hours. Water (10 mL) was added, and the mixture was extracted with EtOAc (3 (10 mL). The combined organic phase was washed with water (30 mL), brine (30 mL), and dried (Na₂SO₄). After evaporation of the volatiles, the residue was purified by flash column chromatography (sillica, hexanes:EtOAc 2:1 (1:1) to yield epothilone **3** as a white solid (7.2 mg, 72%); TLC *R_{f}* = 0.29 (silica, hexanes:EtOAc 1:1); [α]*_{D}*22 -53 (*c* 0.51, CH₂Cl₂); IR (film) *v*ₘₐₓ 3472 (br), 2967, 2920, 1731, 1684, 1461, 1420, 1378, 1249, 1143, 1032, 973, 879, 732, 667 cm⁻¹; MALDI-FTMS *m*/*z* 562.2267 (MNa⁺), calcd for C₂₇H₄₁NO₆S₂Na 562.2267.

### Construction of aldehyde 32

**Alcohol 23.** To a solution of cyclopropyl alcohol **22** (4.08 g, 24 mmol) (Charette, A. B.; et al. J. Am. Chem. Soc. 1998, 120,11943-11952) in DMF (40 mL) was added sodium hydride (1,45 g, 36 mmol, 60% in mineral oil) portionwise with stirring at 0 °C. After stirring for 0.5 h at 25 °C, the mixture was cooled to 0 °C, benzyl bromide (4.3 mL, 36 mmol) was added over 2 min, and stirring was continued for 12 h at 25°C The reaction was quenched with NH₄Cl (sat., 50 mL), the mixture was extracted with EtOAc (3 (50 mL) and the combined extract was washed with brine (2 (100 mL), dried (Na₂SO₄) and evaporated. The residue was dissolved in CH₂Cl₂:MeOH 4:1 (60 mL), and the solution was ozonized (100 L/h, ca. 5 g O₃/h) at -78 °C for 21 min. **(*****NOTE:** Longer reaction times must be avoided to prevent oxidation of the benzyl ether to the corresponding benzoate.)* Excess ozone was removed by flushing with N₂ for 1 min, and then NaBH₄ (2.75 g, 73 mmol) was added in small portions **(*****CAUTION!** Exothermic!*) followed by methanol (20 mL). The mixture was warmed to 25 °C over 1 hour, and the reaction was quenched by the addition of NH₄Cl (sat., 20 mL). The mixture was extracted with CH₂Cl₂ (2 (50 mL), and the combined extract was washed with brine (100 mL), dried (Na₂SO₄) and evaporated. The residue was purified by flash chromatography (silica, hexanes:EtOAc 5:2) to yield 23 as a yellow oil (5.07 g, 89%). TLC *R_{f}* = 0.20 (silica, hexanes:EtOAc 3:1); [α]*_{D}*22 -7.5 (c 1.76, CHCl₃); IR (film) *v*ₘₐₓ 3390 (br), 2933, 2859, 1452, 1070, 739, 698 cm⁻¹; MALDI-FTMS *m*/*z* 257.1519 (MNa⁺), calcd for C₁₅H₂₂O₂Na 257.1512.

**Iodide 24.** To a solution of cyclopropyl alcohol **23** (10.08 g, 43.0 mmol) in dry CH₂Cl₂ (100 mL) at 0 °C was added methanesulfonyl chloride (4.2 mL, 54 mmol) followed by triethylamine (9.0 mL, 65 mmol) dropwise. A white precipitate started to form immediately. The mixture was stirred at 25 °C for 1 hour, then NH₄Cl (sat., 50 mL) and water (50 mL) were added and the phases were separated. The aqueous phase was extracted with EtOAc (100 mL), and the combined organic phase was washed with brine, dried (Na₂SO₄) and evaporated. The residue was dissolved in dry acetone (200 mL), and sodium iodide (19.3 g, 129 mmol) was added. The initially almost clear solution was refluxed for 40 min, during which time a white precipitate formed. Water (100 mL) was added and the mixture was extracted with ether (500 + 250 mL). The combined extract was dried and evaporated, and the residue was purified by flash chromatography (silica, hexanes:EtOAc 5:1) to yield **24** as a colorless oil (14.16 g, 95%). TLC R_{f}= 0.66 (silica, hexanes:EtOAc 5:1); [α]_{D}22 -16 (c 2.05, CHCl₃); IR (film) *v*ₘₐₓ 2916, 2848, 1453, 1217, 1098, 1073, 735, 697 cm⁻¹; ESI-MS *m*/*z* 367 (MNa⁺), calcd for C₁₅H₂₁IONa 367.

**Hydrazone 26.** A solution of LDA was prepared by adding *n*-BuLi (13.1 mL, 21.0 mmol, 1.6 M in hexanes) to diisopropylamine (2.94 mL, 21.0 mmol) in THF (10 mL) at -78 °C, then warming the solution to 0 °C, and stirring for 10 min. To this LDA solution was added propionaldehyde SAMP hydrazone **25** (3.32 g, 19.5 mmol) (Nicolaou, K. C., et al., J. Am. Chem. Soc. 1997, 119, 7974-7991; Enders, D. Aymmetric Synth. 1984, 3, 275-339; and Enders, D., et al., Synthesis 1996, 1403-1418), and the mixture was stirred for 6 h at 0 °C, during which time a white precipitate formed. The mixture was cooled to -98 °C (MeOH/N₂(I) bath) and a solution of iodide **24** (5.16 g, 15.0 mmol) in THF (20 mL) was added over 0.5 hour. The reaction mixture was then allowed to warm to -10 °C over 14 hours, and then the reaction was quenched with NH₄Cl (sat., 10 mL). The mixture was extracted with EtOAc (100 mL + 2 (50 mL), the combined extract was dried (Na₂SO₄) and evaporated, and the residue was purified by flash chromatography (silica, hexanes:EtOAc 6:1 (4:1) to yield hydrazone **26** as a yellow oil (4.88 g, 84%). TLC *R_{f}* = 0.38 (silica, hexanes:EtOAc 5:1); [α]*_{D}*22 -61 (c 1.45, CHCl₃); IR (film) Vₘₐₓ 2926, 1454, 1097, 736, 697 cm⁻¹; MALDI-FTMS *m*/*z* 387.3008 (MH⁺), calcd for C₂₄H₃₉N₂O₂ 387.3006.

**Aldehyde 17.** A solution of hydrazone **26** (3.82 g, 9.9 mmol) in iodomethane (10 mL) was heated at 60 °C (reflux condenser) for 3 hours, and was then cooled to 25 °C. Excess iodomethane was evaporated and traces removed under oil pump vacuum. The residual yellow syrup was vigorously stirred with 3 N HCl (190 mL) and pentane (190 mL) for 3 h at 25 °C, the phases were separated, and the aqueous phase was extracted with pentane (100 mL). The combined organic phase was dried (Na₂SO₄, NaHCO₃) and evaporated to yield aldehyde **17** as a yellow oil (2.38 g, 88%), [α]*_{D}*22 +2 (c 1,3, CHCl₃); IR (film) *v*ₘₐₓ 2931, 2856, 1724, 1454, 1095, 1074, 736, 698 cm⁻¹; MALDI-FTMS m/z 297,1830 (MNa⁺), calcd for C₁₈H₂₆O₂Na 297.1825.

Due to the configurational lability at C8 (epothilone numbering), the aldehyde should be used immediately in the next step. The dr at C8 was estimated as follows: A sample of **17** was treated with excess NaBH₄ in methanol for 10 min. The reaction was quenched with NH₄Cl (sat.), the mixture was extracted with EtOAc, and the extract was dried (Na₂SO₄) and evaporated. The residue was treated with (*R*)-(-)-MTPACI (2-3 equiv.), excess triethylamine and 4-DMAP in CH₂Cl₂ for 3 hours. Purification by preparative TLC yielded a sample of the (*S*)-MTPA ester, which by ¹H NMR analysis showed a dr = 97:3, with the correct absolute stereochemistry at C8 as the major isomer (Tsuda, M., et al., J. Org. Chem. 2000, 65, 1349-1352). Analogous results were obtained by using (S)-(+)-MTPACI.

**Aldol product 27.** A solution of LDA was prepared by adding *n*-BuLi (7.5 mL, 12 mmol, 1.6 M in hexanes) to diisopropylamine (1.68 mL, 12 mmol) in THF (12 mL) at -78 °C, then warming the solution briefly to 0 °C, and finally cooling back to -78 °C. A solution of ketone **19** (4.63 g, 11.5 mmol) (Nicolaou, K. C., et al., J. Am. Chem. Soc. 1997, 119, 7974-7991) in THF (12 mL) was added dropwise over 2 min, and the mixture was stirred for 1 h at -78°C and then for 0.5 h at -40 °C. It was again cooled to -78 °C, and a solution of aldehyde **17** (1.37 g, 5.0 mmol) in THF (25 mL), pre-cooled to -78 °C, was added via cannula over 1 min, taking care to ensure minimal warming during transfer. The mixture was stirred for 5 min, and the reaction was then quenched by rapid injection of a solution of AcOH (1,4 mL) in THF (4.2 mL). After 5 min at -78 °C, the mixture was warmed to 25 °C and partitioned between NH₄Cl (sat., 50 mL) and ether (50 mL). The aqueous phase was extracted with ether (2 (50 mL), the combined extract was dried (Na₂SO₄) and evaporated, and the residue was purified by flash chromatography (silica, hexanes:ether 20:1 ( 6:1) to yield recovered ketone **19** (1.71 g, 4.25 mmol) followed by the aldol product **27** in diastereomerically pure form (2.73 g, 81%). TLC *R_{f}* = 0.34 (silica, hexanes:EtOAc 5:1); [α]*_{D}*22 -40 (c 1.0, CHCl₃); IR (film) *v*ₘₐₓ 3502 (br), 2954, 2928, 2856, 1681, 1472, 1255, 1098, 836, 776 cm⁻¹; MALDI-FTMS m/z 699.4796 (MNa⁺), calcd for C₃₉H₇₂O₅Si₂Na 699.4816.

**Alcohol 28**. A solution of aldol product **27** (2.71 g, 4.0 mmol) and 2,6-lutidine (1,40 mL, 12 mmol) in CH₂Cl₂ (25 mL) was cooled to -20 °C and then TBSOTf (1.84 mL, 8.0 mmol) was added dropwise. The mixture was stirred for 1 h at -20 °C and the reaction was then quenched by the addition of NH₄Cl (sat., 25 mL). The mixture was warmed to 25 °C, the phases were separated and the aqueous phase was extracted with CH₂Cl₂ (25 mL) and ether (25 mL). The combined organic phase was dried (Na₂SO₄) and evaporated, and the residue was filtered through a plug of silica eluting with hexane:ether 10:1. The filtrate was evaporated and the resulting crude silyl ether (3.14 g, 4.0 mmol, 99%) was dissolved in THF (40 mL). To this was added a cold (0 °C) solution of HF-pyridine complex (6.4 mL) and pyridine (18 mL) in THF (32 mL) at 0 °C (this solution was prepared by slowly adding the HF·pyridine complex to a solution of pyridine in THF at 0 °C; ***CAUTION!** HF·pyridine is highly corrosive. The addition of HF·pyridine to the pyridine-THF solution is highly exothermic, and must be done with stirring and cooling in ice bath to prevent splashing*), and the resulting solution was stirred at 25°C for 4 hours. The mixture was diluted with EtOAc (100 mL), placed in an ice bath, and quenched by the careful addition of NaHCO₃ (sat., 100 mL) and as much solid NaHCO₃ as needed to ensure complete neutralization (***CAUTION!** Foaming!).* The mixture was extracted with EtOAc (3 (100 mL), and the combined extract was dried (Na₂SO₄) and evaporated, and the residue was purified by flash chromatography (silica, hexanes:EtOAc 5:1) to yield **28** as a colorless oil (2.40 g, 89%). TLC R_{f} = 0.39 (silica, hexanes:EtOAc 5:1); [α]_{D}22 -26 (c 1.1, CHCl₃); IR (film) *v*ₘₐₓ 3458 (br), 2929, 2856, 1693, 1472, 1462, 1255, 1093, 986, 836, 775 cm⁻¹; MALDI-FTMS m/z 699.4807 (MNa⁺), calcd for C₃₉H₇₂O₅Si₂Na 699.4816,

**Ester 29.** The alcohol **28** (2.40 g, 3.5 mmol), Dess-Martin periodinane (3.75 g, 8.8 mmol), NaHCO₃ (0.74 g, 8.8 mmol) and water (76 µL, 4.2 mmol) were mixed in CH₂Cl₂ (80 mL), and the resulting suspension was stirred for 1 hour. The mixture was diluted with ether (200 mL), water (100 mL) and NaHCO₃ (sat., 100 mL), and was then filtered. The phases were separated and the aqueous phase was extracted with ether (2 (100 mL). The combined extract was dried (Na₂SO₄) and evaporated, and the residue was filtered through a plug of silica eluting with hexanes:EtOAc 6:1. The filtrate was evaporated and the resulting crude aldehyde (2.15 g, 3.2 mmol, 90%) was dissolved in a mixture of THF (80 mL), *t*-BuOH (145 mL) and 2-methyl-2-butene (25 mL). To this solution was added a solution of NaH₂PO₄ (0.95 g, 6.7 mmol) and NaClO₂ (1.14 g, 10 mmol) in water (31 mL), and the resulting mixture was stirred vigorously for 1 hour. The volatiles were removed by evaporation, and the residue was partitioned between EtOAc (100 mL) and brine (100 mL). The phases were separated and the aqueous phase was extracted with EtOAc (3 (100 mL). The combined extract was dried (Na₂SO₄) and evaporated, and the residue was dissolved in DMF (5 mL) and evaporated again to remove traces of *t*-BuOH. The so obtained crude acid (2.4 g, ca. 3.2 mmol >100%,) was again dissolved in DMF (10 mL), to which 2-(trimethylsilyl)ethanol (1.83 mL, 12,7 mmol), EDC (0,92 g, 4.8 mmol), and 4-DMAP (40 mg, 0.33 mmol) were added. The resulting suspension was stirred for 14 hours, after which time a clear solution was obtained. Water (10 mL) was added and the mixture was extracted with ether (3 (50 mL). The combined extract was washed with water-brine mixture (100 + 100 mL), dried (Na₂SO₄) and evaporated. The residue was purified by flash chromatography (silica, hexanes:EtOAc 10:1) to yield ester **29** as a viscous, pale yellow oil (2.08 g, 74%). TLC *R_{f}*= 0.57 (silica, hexanes:EtOAc 10:1); [α]_{D}22 -33 (c 1.2, CHCl₃); IR (film) *v*ₘₐₓ 2954, 2930, 2856, 1735, 1695, 1472, 1385, 1252, 1090, 988, 836, 776 cm⁻¹; MALDI-FTMS *m*/*z* 813-5315 (MNa⁺), calcd for C₄₄H₈₂O₆Si₃Na 813.5311.

**Aldehyde 30.** To a solution of benzyl ether **29** (2.08 g, 2.63 mmol) in EtOH:EtOAc 1:1 (50 mL) was added 20% Pd(OH)₂ on carbon (2.1 g, 60% moisture), and the mixture was hydrogenated for 1 hour. It was then filtered through celite to remove the catalyst, the filtrate was evaporated, and the residue was co-evaporated with benzene to remove traces of EtOH. The resulting crude alcohol (1.89 g, ca. 2.6 mmol, >100%) was dissolved in CH₂Cl₂ (60 mL), Dess-Martin periodinane (2.76 g, 6.5 mmol), NaHCO₃ (0.55 g, 6.5 mmol) and water (56 µL, 3.1 mmol) were added, and the resulting suspension was stirred for 1 hour. The mixture was diluted with ether (150 mL), water (75 mL) and NaHCO₃ (sat., 75 mL), and was then filtered. The phases were separated and the aqueous phase was extracted with ether (2 (75 mL). The combined extract was dried (Na₂SO₄) and evaporated, and the residue was purified by flash chromatography (silica, hexanes:EtOAc 95:1) to yield aldehyde 30 as a viscous oil (1.55 g, 84%). TLC *R_{f}*= 0.24 (silica, hexanes:EtOAc 15:1); [α]*_{D}*22 - 47 (c 1.3, CHCl₃); IR (film) *v*ₘₐₓ 2954, 2856, 1734, 1703, 1251, 1173, 1084, 988, 837, 776 cm⁻¹; MALDI-FTMS *m*/*z* 721.4671 (MNa⁺), calcd for C₃₇H₇₄O₆Si₃Na 721,4685.

**Enol ether 31.** To a suspension of MeOCH₂PPh₃Cl (3.09 g, 9.0 mmol) in THF (20 mL) at 0 °C was added NaHMDS (8.5 mL, 8.5 mmol, 1 M in THF) dropwise. A red color developed. The mixture was stirred at 0 °C for 0.5 h and it was then cooled to - 40 °C. A solution of aldehyde 30 (2.12 g, 3.0 mmol) in THF (7 mL) was added, and the mixture was allowed to warm to -10 °C over 2 hours. The reaction was quenched with NH₄Cl (sat., 15 mL), the phases were separated, and the aqueous phase was extracted with EtOAc (2 (75 mL). The combined extract was dried (Na₂SO₄) and evaporated, and the residue was purified by flash chromatography (silica, hexanes:EtOAc 30:1) to yield enol ether 31 as a colorless, viscous oil (1.85 g, 84%, olefin *cis:trans ca.* 1:1 by ¹H NMR). TLC *R_{f}* = 0.23 (silica, hexanes:EtOAc 30:1); [α]*_{D}*22 -36 (*c* 1.2, CHCl₃); IR (film) *v*ₘₐₓ 2954, 2930, 2856, 1735, 1695, 1251, 1171, 1105, 988, 836, 776 cm⁻¹; MALDI-FTMS *m*/*z* 749.4996 (MNa⁺), calcd for C₃₉H₇₈O₆Si₃Na 749.4998.

**Aldehyde 32.** To a solution of enol ether 31 (847 mg, 1.16 mmol) in dioxane:water 9:1 (12 mL) was added pyridinium para-toluenesulfonate (2.34 g, 9.31 mmol) and the mixture was stirred at 70 °C until TLC indicated the completion of the reaction (6-10 h). The reaction was then quenched with NaHCO₃ (sat., 15 mL), and the mixture was extracted with EtOAc (3 (50 mL). The combined extract was dried (Na₂SO₄) and evaporated, and the residue was purified by flash chromatography (silica, hexanes:EtOAc 15:1) to yield **32** as a colorless, viscous oil (681 mg, 82%). TLC R_{f} = 0-29 (silica, hexanes:EtOAc 15:1); [α]*_{D}*22 -34 (c 1.0, CHCl₃); IR (film) Vₘₐₓ 2954, 2856, 1731, 1695, 1251, 1086, 988, 836, 776 cm⁻¹; MALDI-FTMS *m*/*z* 735.4823 (MNa⁺), calcd for C₃₈H₇₆O₆Si₃Na 735.4842.

### Construction of vinyl iodides 20e

### Sonogashira coupling of aryl bromdes (38) with propyne (general procedure).

To a briefly deoxygenated (Ar bubbling) solution of the aryl bromide 38 (3.5 mmol) in DMF (3 mL) and diisopropyl amine (2.5 mL) were added Pd(PPh₃)₂Cl₂ (25 mg, 36 µmol) and Cul (13 mg, 70 µmol) under Ar(g), and then the inert atmosphere was replaced by propyne (1 atm, balloon). The mixture was stirred at 25 °C for 3 hours. During this time, a precipitate formed, and the reaction mixture turned dark brown. Water (15 mL) was added, the mixture was extracted with EtOAc, and the combined extract was dried (Na₂SO₄) and evaporated. The pure 1-arylpropyne was obtained by flash chromatography (silica, hexane:EtOAc mixtures).

**Sonogashira coupling product from 38.** Brown oil (97%); TLC *R_{f}* = 0.21 (silica, hexanes:EtOAc 5:1); IR (film) *v*ₘₐₓ 2908, 2226, 1567, 1531, 1461, 1431, 1361, 1108, 1008, 832 cm⁻¹; MALDI-FTMS *m*/*z* 164.0527 (MH⁺), calcd for C₉H₁₀NS 164.0528.

**Hydrostannylation-iodination (general procedure).** This is an adaption of the previously reported procedure (Betzer, J.-F., et al., Tetrahedron Lett. 1997, 38, 2279-2282). To a solution of hexabutylditin (10.1 mL, 20 mmol) in dry THF (40 mL) at -78 °C was added *n*-BuLi (12.9 mL, 20 mmol, 1.55 M in hexanes), and the resulting clear solution was stirred at -40 °C for 30 min. It was then transferred via cannula to a suspension of CuCN (0.90 g, 10 mmol) in THF (2 mL) at -78 °C. A clear yellow solution formed, and it was stirred for 5 min at -40 °C before being re-cooled to -78 °C. Then dry methanol (23 mL, 0.57 mol) was added to yield a red solution, which was stirred at -40 °C for 15 min, after which a solution of the arylpropyne (5.0 mmol) in THF (5 mL) was added. The orange-red solution was stirred at -10°C overnight (some Cu and/or Cu²⁺ salts precipitate), then cooled to -20 °C, followed by the addition of methanol (10 mL). After 15 min at -20°C, water (10 mL) was added, and stirring was continued for another 15 min, while warming to 25°C. The mixture was extracted with ether, and the organic phase was washed with brine, dried (Na₂SO₄) and evaporated. Flash chromatography (silica, hexanes:EtOAc mixtures) yielded the intermediate vinylstannane, which was dissolved in CH₂Cl₂ (5 mL). A solution of iodine (1.05 equiv.) in CH₂Cl₂ (40 mL per g I₂) was then added dropwise to this solution at 0 °C. After the last few drops, the color of I₂ persisted, and the reaction was allowed to continue for another 5 min at 0 °C. Then the solvent was evaporated and the residue was dissolved in ether. KF (1 M solution in water, 3 equiv.) and Na₂S₂O₃ (sat., 10 mL per mmol substrate) were added, and the mixture was stirred for 15 min at 25 °C during which time a white precipitate formed. The mixture was filtered through celite, and the organic phase was dried (Na₂SO₄) and evaporated. The residue was purified by flash chromatography (silica, hexanes:EtOAc mixtures) to yield the desired vinyl iodide.

**Vinyl iodide 20e.** The intermediate vinyl stannane is readily protodestannylated; therefore, flash chromatography of this intermediate must be performed using hexanes:EtOAc:Et₃N 50:1:1 as eluent, and the so obtained vinylstannane contained other butyl tin compounds. Following the general procedure, the mixture was treated with enough I₂ that the brown color persisted at the end of the addition (ca. 2 equiv. of I₂). After flash chromatography (hexanes:EtOAc 50:1), vinyl iodide **20e** was obtained as a yellow oil (74%). TLC R_{f}= 0.41 (silica, hexanes:EtOAc 50:1); IR (film) *v*ₘₐₓ 3102, 2923, 1620, 1423, 1300, 1065, 1035, 964, 863, 723, 562 cm⁻¹; MALDI-FTMS m/z 297.9215 (MH⁺), calcd for C₇H₉INS₂ 297.9216.

### Synthesis of epothilone analogues 8-144.

**Nozaki-Hiyama-Kishi coupling of aldehydes (34, 40) with vinyl stannanes (20a-g) (general procedure).** To a briefly vacuum-degassed solution of aldehyde **32** (107 mg, 0.15 mmol), the requisite vinyl iodide **20** (0.45 mmol), and 4-*tert*-butylpyridine (665 µL, 4.5 mmol) in DMSO (3 mL) were added anhydrous CrCl₂ (184 mg, 1.5 mmol) and anhydrous NiCl₂ (4 mg, 0.03 mmol). The mixture was stirred at 25 °C for 3 hours, after which another portion of vinyl iodide (0.45 mmol) was added, and stirring was continued for a further 3 hours. This was repeated one more time, after which stirring was continued overnight. The reaction was then quenched with water (5 mL), pyridine (1 mL) was added to prevent Cr-product complexes from being extracted into the water phase, and the mixture was extracted with EtOAc (3 (25 mL). The combined extract was washed with brine (2 (100 mL), dried (Na₂SO₄) and evaporated. Flash chromatography (silica, hexanes:EtOAc mixtures) yielded the coupling product, in most cases inseparable from excess 4-*tert*-butylpyridin.

**Product from 20e and 32.** Yellow glass (78%, ca. 1;1 mixture of C15 epimers). TLC *R_{f}* = 0.40 (silica, hexanes:EtOAc 5:1); [α]*_{D}*22 -28 (c 2.0, CHCl₃); IR (film) Vₘₐₓ 3416 (br), 2929, 2856, 1732, 1694, 1472, 1251, 1037, 988, 836, 776 cm⁻¹; MALDI-FTMS m/z 906.5021 (MH⁺), calcd for C₄₅H₈₅NO₆S₂Si₃Na 906.5018.

**TBAF deprotection (general procedure).** The product mixture from the Nozaki-Hiyama-Kishi coupling was dissolved in THF (1.5 mL), and TBAF (1 M in THF, 0.30 mL, 0.30 mmol) was added at 0 °C. After 1 h at 0 °C, another portion of TBAF (0.30 mL, 0.30 mmol) was added, and the mixture was stirred at 25 °C for 1 hour. The reaction was quenched with NH₄Cl (sat., 5 mL), and the mixture was extracted with EtOAc (4 (20 mL). The combined extract was dried (Na₂SO₄) and evaporated, and the residue was purified by flash chromatography (silica, hexanes:EtOAc mixtures) to yield the desired hydroxy acid as a ca. 1:1 mixture of C15 epimers (inseparable at this stage).

**Hydroxy acid 41e.** Yellow solid (79%, *ca*. 1:1 mixture of C15 epimers). TLC *R_{f}*= 0.37 (silica, hexanes:EtOAc 2:1); [α]*_{D}*22 -23 (*c* 2.3, CHCl₃); IR (film) *v*ₘₐₓ 3356 (br), 2929, 2856, 1712, 1472, 1253, 1085, 1038, 988, 836, 776 cm⁻¹; MALDI-FTMS *m*/*z* 806.4282 (MNa⁺), calcd for C₄₀H₇₃NO₆S₂Si₂Na 806.4315.

**Yamaguchi macrolactonization (general procedure).** To a solution of the hydroxy acid (95 µmol) in dry THF (8 ml) at 0 °C was added triethylamine (79 µl, 0.57 mmol) and 2,4,6-trichlorobenzoyl chloride (40 µl, 0.23 mmol). After stirring at 0 °C for 1 hour, the resulting solution was added over 2 h to a solution of 4-DMAP (26 mg, 0.21 mmol) in toluene (20 mL) at 75 °C using a syringe pump. Stirring was continued at 75 °C for another 1 h after which the toluene was evaporated under reduced pressure. The residue was directly subjected to flash chromatography (silica, hexanes:EtOAc mixtures) to yield the macrolactone and its (15*R*)-epimer, readily separable. In all cases the desired (15S)-epimer eluted *after* the less polar (15*R*)-epimer.

**Macrolactone 43e.** This product was isolated as a crude mixture which was directly subjected to the global desilylation conditions (*vide infra*) without further purification.

**Global desilylation (general procedure).** The macrolactone was dissolved in 20% v/v TFA in CH₂Cl₂, and the solution was kept at 25°C for 3 hours, after which the volatiles were evaporated without heating. The residue was dissolved in EtOAc, and the solution was washed with NaHCO₃ (sat.), dried (Na₂SO₄) and evaporated. Flash chromatography (silica, hexanes:EtOAc mixtures) afforded the pure epothilone.

**Epothilone 12.** Viscous oil (17% from **41e**); TLC *R_{f}*= 0.38 (silica, hexanes:EtOAc 2:1); [α]_{D}22 -52 (*c* 0.50, CHCl₃); IR (film) *v*ₘₐₓ 3490 (br), 2933, 1732, 1686, 1255, 1038, 756 cm⁻¹; MALDI-FTMS *m*/*z* 538,2666 (MH⁺), calcd for C₂₈H₄₄NO₅S₂ 538.2655.

### Example : Soft capsules

5000 soft gelatin capsules, each containing as active ingredient 0.05 g of one of the compounds of formula I named in the preceding examples, are prepared as follows:

| Composition | |
|---|---|
| active ingredient | 250 g |
| Lauroglycol | 2 litres |

Preparation process: The pulverised active ingredient is suspended in Lauroglykol^{®} (propylene glycol laurate, Gattefossé S.A., Saint Priest, France) and ground in a wet pulverizer to a grain size of approximately 1 to 3 µm. Portions each containing 0.419 g of the mixture are then filled into soft gelatin capsules by a capsule filling machine.

### Detailed Description of Figures

Figure 1 shows the structures of selected natural and designed epothilones. Grey boxes indicate compounds synthesized in this study.

Figure 4 shows the retrosynthetic analysis of *trans*-cyclopropyl epothilone B analogue (**12**).

Figure 5 shows the construction of aldehyde **32**. *Reagents and conditions:* (a) See Nicolaou, K. C., et al. ChemBioChem 2001, 2, 69-75; Charette, A. B.; et al. J. Am. Chem. Soc. 1998, 120,11943-11952; (b) NaH (1.5 equiv), BnBr (1.5 equiv), DMF, 0 → 25 °C, 12 h; (c) O₃, CH₂Cl₂:MeOH 4:1, -78 °C, 21 min; then NaBH₄ (3.0 equiv), - 78 → 25 °C, 1 h, 89% for 2 steps; (d) MsCl (1.3 equiv), Et₃N (1.5 equiv), CH₂Cl₂, 25 °C , 1 h; (e) Nal (3.0 equiv), acetone, reflux, 40 min, 95% for 2 steps; (f) LDA (1.4 equiv), **25** (1.3 equiv), THF, 0 °C, 6 h; then **24**, -98 → -10 °C, 14 h, 84%; (g) Mel, 60 °C, 3 h; (h) 3 N HCl:pentane 1:1, 25 °C, 3 h, 88% for 2 steps; (i) LDA (2.4 equiv), **19** (2.3 equiv), THF, -78 °C, 1 h; then -40 °C, 0.5 h; then 17 at -78 °C, 5 min, 81 %; (j) TBSOTf (2.0 equiv), 2,6-lutidine (3.0 equiv), PH₂Cl₂, -20 °C, 1 h; (k) HF-py, pyridine, THF, 25 °C, 4 h, 89% for 2 steps; (I) DMP (2.5 equiv), NaHCO₃ (2.5 equiv), H₂O, CH₂Cl₂, 25 °C, 1 h; (m) NaClO₂ (3.1 equiv), NaH₂PO₄ (2.1 equiv), 2-methyl-2-butene (74 equiv), *t*-BuOH, THF, H₂O, 25 °C, 1 h; (n) 2-(trimethylsilyl)ethanol (4.0 equiv), EDC (1.5 equiv), 4-DMAP (0.1 equiv), DMF, 25 °C, 14 h, 74% for 3 steps; (o) 20% Pd(OH)₂/C, H₂ (1 atm), EtOH:EtOAc 1:1, 25 °C, 1 h; (p) DMP (2.5 equiv), NaHCO₃ (2.5 equiv), H₂O, CH₂Cl₂, 25 °C, 1 h, 84% for 2 steps; (q) MeOCH₂PPh₃Cl (3.0 equiv), NaHMDS (2.8 equiv), THF, -40 → -10 °C, 2 h, 84%; (r) PPTS (8.0 equiv), dioxane:H₂O 9:1, 70 °C, 6 h, 82%. 4-DMAP = 4-(dimethylamino)pyridine; DME = 1,2-dimethoxy-ethane; DMP = Dess-Martin periodinane; EDC = 1-(3-dimethylaminopropyl)-3-ethylcarbodiimide hydrochloride; HF-py = hydrogen fluoride-pyridine complex; NaHMDS = sodium hexamethyldisilazide; PPTS = pyridinium para-toluenesulfonate; TMSE = 2-trimethylsilylethyl.

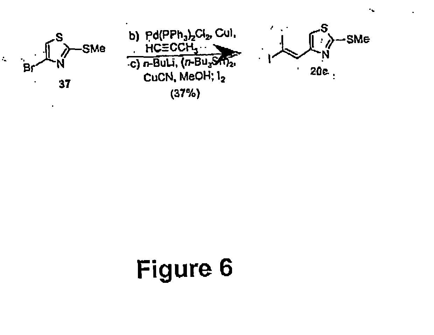

Figure 6 shows the construction of vinyl iodides **20e**. *Reagents and conditions:* (b) Pd(PPh₃)₂Cl₂ (0.01 equiv), Cul (0.02 equiv), HCéCCH₃ (1 atm), DMF, *i*-Pr₂NEt, 25 °C, 3 h, **35:** 96%; (c) (i) *n*-BuLi (4.0 equiv), (*n*-Bu₃Sn)₂ (4.0 equiv), CuCN (2.0 equiv), MeOH, THF, -10 °C, 12 h; (ii) I₂ (1.05 equiv), CH₂Cl₂, 0 °C, 5 min, **20e:** 37% from **37;.** TrCI = triphenylmethyl chloride; 4-DMAP = 4-(dimethylamino)pyridine; MOMCI = chloromethyl methyl ether.

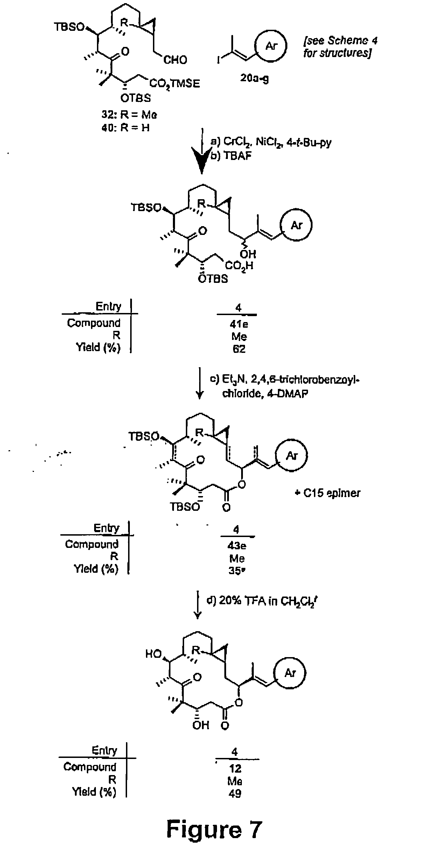

Figure 7 is the synthesis of epothilone analogues **12**. *Reagents and conditions:* (a) CrCl₂ (10 equiv), NiCl₂ (0.2 equiv), 4-*t*-butylpyridine (30 equiv), 20 (3.0 equiv), DMSO, 25 °C, overnight; (b) TBAF (4.0 equiv), THF, 0 °C, 1 h; then 25 °C, 1 h; (c) Et₃N (6.0 equiv), 2,4,6-trichlorobenzoylchloride (2.4 equiv), **41** or **42,** THF, 0 °C, 1 h; then 4-DMAP (2.2 equiv), toluene, 75 °C, 3 h; (d) 20 v/v% TFA in CH₂Cl₂, 25 °C, 3 h (except **43d);** (e) Estimated by ¹H NMR ; (f) Deprotection of **43d:** TMSBr (10 equiv), 4Å MS, CH₂Cl₂, -30 °C, 1 h; then 20 v/v% TFA in CH₂Cl₂, 25 °C, 3 h. TBAF = tetrabutylammonium fluoride; 4-DMAP = 4-(dimethylamino)pyridine; TFA = trifluoroacetic acid; TMSBr = trimethylsilyl bromide; MS = molecular sieves.

Figure 8 illustrates a table disclosing the cytotoxicity of epothilones **1** through **14** and paclitaxel against 1A9 human ovarian carcinoma cells and β-tubulin mutant cell lines selected with paclitaxel or epothilone A. The anti-proliferative effects of the tested compounds against the parental 1A9 and the paclitaxel- and epothilone-selected drug-resistant clones (PTX10, PTX22 and A8, respectively) were assessed in a 72 h growth inhibition assay using the SRB (sulforhodamine-B) assay (Skehan, P.; et al. J. Natl. Cancer Inst. 1990, 82, 1107-1112). IC₅₀ values for each compound are given in nM and represent the mean of 3-9 independent experiments Å standard error of the mean. Relative resistance (RR) is calculated as an IC₅₀ value for each resistant sub-line divided by that for the parental cell line (1A9). CP = cyclopropyl; py = 5-methylpyridine side chain; pyOH = 5-hydroxymethylpyridine side chain; 5tmpy = 5-thiomethylpyridine side chain; 6tmpy = 6-thiomethylpyridine side chain; tmt = 2-thiomethyl thiazole side chain.

Figure 9 illustrates a table disclosing the tubulin polymerization potency and cytotoxicity of epothilones **1-8, 10-14,** and paclitaxel against human epidermoid cancer cell lines. (a) The extent of porcine tubulin polymerization (TP) by 4 µM compound was quantified relative to the effect of 25 µM epothilone B (which was defined as 100%) as described (Nicolaou, K. C.; et al. Chem. Biol. 2000, 7, 593-599). (b) Drug concentration required for maximal inhibition of cell growth (IC₅₀ values given in nM) was assessed after a 96 hour drug exposure by quantification of cell mass using a protein dye method as described (Meyer, T.; et al. Int. J. Cancer 1989, 43, 851-856), KB-31: epidermoid Taxol^{®}-sensitive cells, KB-8511: epidermoid Taxol^{®}-resistant cells (due to Pgp overexpression). Relative resistance (RR) was calculated by dividing the IC₅₀ value for the resistant cell line by that of the sensitive cell line. (c) Data from ref. 3 (%TP values for Taxol^{®}, Epo A and Epo B were 49, 69 and 90, respectively). CP = cyclopropyl; py = 5-methylpyridine side chain; pyOH = 5-hydroxymethylpyridine side chain; 5tmpy = 5-thiomethylpyridine side chain; 6tmpy = 6-thiomethylpyridine side chain; tmt = 2-thiomethyl thiazole side chain.

Figure 10 illustrates a table disclosing binding affinities of epothilone analogues to the taxoid binding site of microtubules. (a) The binding of the different ligands to the taxoid site of microtubules was measured by the displacement of a fluorescent Taxol^{®} derivative (Flutax-2) from its binding site (Figure 2) (Diaz, J. F.; et al. J. Biol. Chem. 2000, 275, 26265-26276). The Flutax-2 displacement isotherm of each ligand was measured at least twice with a fluorescence polarization microplate reader in a modified procedure from the previous report (Andreu, J. M.; Barasoain, I. Biochemistry 2001, 40, 11975-11984). Cross-linked stabilized microtubules which had been stored under liquid nitrogen were employed. The binding constant of the reference ligand Flutax-2 was measured by centrifugation and fluorescence anisotropy, at each temperature (Diaz, J. F.; et al. J. Biol. Chem. 2000, 275, 26265-26276). The resulting reference value was 2.2 (10⁷ M⁻¹ at 37 °C. (b) The equilibrium dissociation constants (Kd) are given in nM. (c) The standard binding free energy changes (DG⁰ₐₚₚ) are given in kJ mol⁻¹.

## Claims

1. A compound represented by formula I:

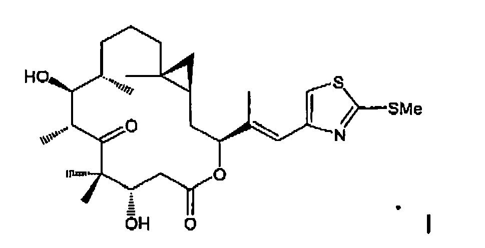

2. A pharmaceutical composition comprising a pharmaceutically acceptable carrier or diluent and a compound according to claim 1, or a pharmaceutically acceptable acid or base addition salt thereof, where possible.

3. Use of a compound according to claim 1 for the preparation of a medicament for the treatment of a proliferative disease.

4. A process for the preparation of a compound according to claim 1,
wherein a compound of the formula VI 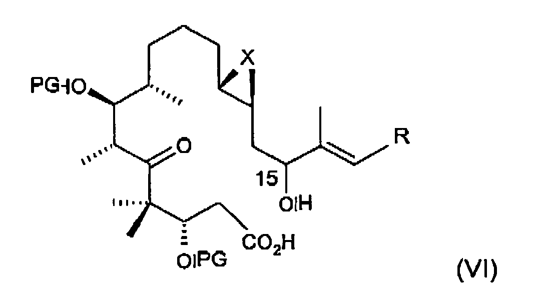 wherein X is CH₂and R is 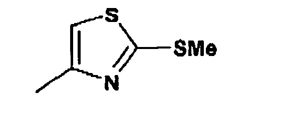 and PG is a protecting group for a hydroxy function,
in a first step is condensed by a esterification reaction, optionally in the presence of a catalyst,
and in a second step the protecting group is detached thus furnishing a lactone of formula I.

## Patentansprüche

1. Verbindung der Formel I

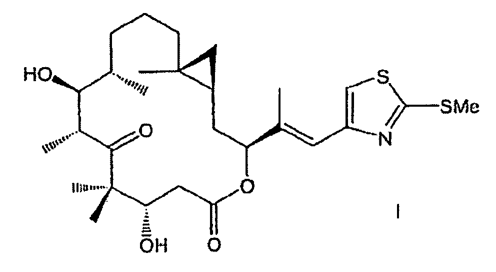

2. Pharmazeutische Zusammensetzung, umfassend einen pharmazeutisch akzeptablen Träger oder ein pharmazeutisch akzeptables Verdünnungsmittel oder eine Verbindung nach Anspruch 1 oder, falls möglich, ein pharmazeutisch akzeptables Säureadditionssalz oder Basenadditionssalz hiervon.

3. Verwendung einer Verbindung nach Anspruch 1 zur Herstellung eines Arzneimittels für die Behandlung einer proliferativen Krankheit.

4. Verfahren zur Herstellung einer Verbindung nach Anspruch 1, worin eine Verbindung der Formel VI 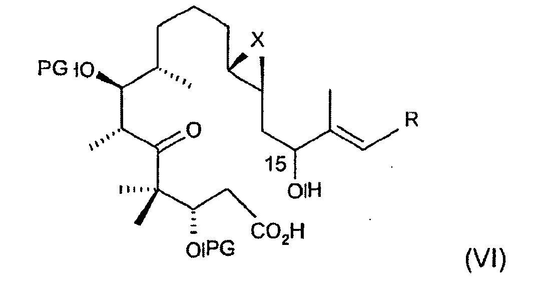 worin X für CH₂ steht und R für 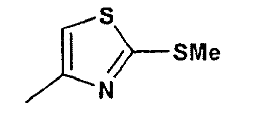 steht und PG eine Schutzgruppe für eine Hydroxyfunktion ist,
in einer ersten Stufe durch eine Veresterungsreaktion, optional in Gegenwart eines Katalysators, kondensiert wird und
in einer zweiten Stufe die Schutzgruppe abgespalten und so ein Lacton der Formel I gebildet wird.

## Revendications

1. Composé représenté par la formule I :

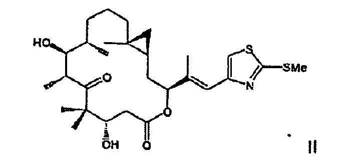

2. Composition pharmaceutique comprenant un véhicule ou un diluant acceptable sur le plan pharmaceutique et un composé selon la revendication 1, ou un sel d'addition d'acide ou de base de celui-ci acceptable sur le plan pharmaceutique, si possible.

3. Utilisation d'un composé selon la revendication 1 pour la préparation d'un médicament destiné au traitement d'une maladie proliférative.

4. Procédé de préparation d'un composé selon la revendication 1, dans lequel un composé de formule VI 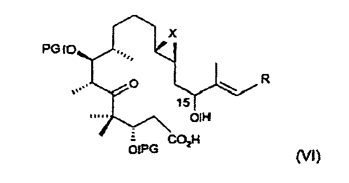 dans laquelle C est CH₂ et R est 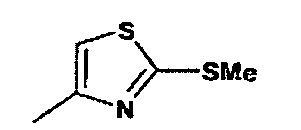 et PG est un groupe protecteur d'une fonction hydroxy,
est condensé lors d'une première étape par une réaction d'estérification, éventuellement en présence d'un catalyseur,
et lors d'une seconde étape, le groupe protecteur est détaché, fournissant alors une lactone de formule I.
